# EUROPEAN PATENT SPECIFICATION

(11) **EP 2 931 275 B1**
(45) Date of publication and mention of the grant of the patent: **01.06.2022**
(21) Application number: 13805376.4
(22) Date of filing: 13.12.2013
(51) Int. Cl.: A61K 31/439, A61P 11/06, A61P 11/08, A61K 9/00

(54) **ACLIDINIUM FOR USE IN INCREASING PHYSICAL ACTIVITY IN DAILY LIFE IN A PATIENT SUFFERING FROM CHRONIC OBSTRUCTIVE PULMONARY DISEASE**
ACLIDINIUM ZUR VERWENDUNG DER VERBESSERTEN KÖRPERLICHEN AKTIVITÄT IM ALLTAG VON PATIENTEN MIT CHRONISCH OBSTRKTIVER PULMONALER ERKRANKUNG
ACLIDINIUM ET SON UTILISATION POUR L'AUGMENTATION DE L'ACTIVITE PHYSIQUE DANS LA VIE QUOTIDIENNE DES PATIENTS SOUFFRANT D'UNE MALADIE PULMONAIRE OBSTRUCTIVE CHRONIQUE

(30) Priority: 17.12.2012 EP 12382507; 10.01.2013 US 201361750952 P
(43) Date of publication of application: 21.10.2015
(73) Proprietor: Almirall S.A., 08022 Barcelona (ES)
(72) Inventor: JARRETA FERNANDEZ, Diana, E-08980 Barcelona (ES); GARCIA GIL, Maria Esther, E-08980 Barcelona (ES)
(74) Representative: J A Kemp LLP
(86) International application number: PCT/EP2013/076606
(87) International publication number: WO 2014/095663

(56) References cited:
- EP-A1- 2 100 599
- FRANOIS MALTAIS ET AL: "Aclidinium bromide improves exercise endurance and lung hyperinflation in patients with moderate to severe COPD", RESPIRATORY MEDICINE, BAILLIERE TINDALL, LONDON, GB, vol. 105, no. 4, 16 November 2010 (2010-11-16), pages 580-587, XP028154207, ISSN: 0954-6111, DOI: 10.1016/J.RMED.2010.11.019 [retrieved on 2010-11-23]
- H. CHRYSTYN ET AL: "The Genuair inhaler: a novel, multidose dry powder inhaler", INTERNATIONAL JOURNAL OF CLINICAL PRACTICE, vol. 66, no. 3, 16 February 2012 (2012-02-16), pages 309-317, XP055039099, ISSN: 1368-5031, DOI: 10.1111/j.1742-1241.2011.02832.x
- WATZ H ET AL: "Physical activity in patients with COPD.", THE EUROPEAN RESPIRATORY JOURNAL : OFFICIAL JOURNAL OF THE EUROPEAN SOCIETY FOR CLINICAL RESPIRATORY PHYSIOLOGY FEB 2009, vol. 33, no. 2, February 2009 (2009-02), pages 262-272, XP002691101, ISSN: 1399-3003 cited in the application
- VAN GESTEL ARNOLDUS J R ET AL: "Predicting daily physical activity in patients with chronic obstructive pulmonary disease.", PLOS ONE 2012, vol. 7, no. 11, 2 November 2012 (2012-11-02), page e48081, XP002691102, ISSN: 1932-6203
- None

## Description

### Field of the Invention

The invention relates to a novel use of aclidinium, which can be advantageously used to increase physical activity in daily life in respiratory patients.

### Background of the Invention

Respiratory diseases, such as asthma and chronic obstructive pulmonary disease (COPD), are a significant global health problem, with an increasing incidence throughout the world. They are usually characterised by an inflammatory dysfunction of the airways which results in bronchoconstriction.

In asthma inflammation is driven by exposure to a variety of triggers, including allergens and viruses, which activate components of both the innate and acquired immune responses. In COPD inflammation occurs primarily because of exposure to noxious particles and gases, in particular to cigarette smoke. Rather than a single pathologic condition, COPD is a term encompassing several disorders, such as chronic bronchitis or emphysema.

According to a scientific statement from the American Heart Association (Thompson, PD et al, Arterioscler Thromb Vasc Biol. 2003; 23: e42-e49), physical activity is defined as "any bodily movement produced by skeletal muscles that results in energy expenditure beyond resting expenditure". This definition of physical activity includes activities such as activities of daily living, sports, and activities for personal fulfilment (Gimeno-Santos et al, Health and Quality of Life Outcomes, 2011; 9: 86).

In certain groups of respiratory patients, for example those with a more severe degree of the disease, physical activity is significantly reduced. This reduction of physical activity may contribute to a further deterioration of the health status of the patients and result in an exacerbation of the respiratory symptoms and/or the appearance of co-morbidities, such as cardiovascular or metabolic diseases in addition to deconditioning and muscle weakness.

Physical activity can be measured in terms of walking and/or running distance (steps or meters), walking and/or running time (min), standing time (min), sitting time (min), lying time (min), movement intensity during walking and/or running (meters/s2), calories expended or metabolic equivalents, skin temperature, heat flux (amount of heat dissipating from the body), galvanic skin response (electrical conductivity of the skin, which changes in response to sweat and emotional stimuli), among others.

Physical activity monitors are frequently used to estimate levels of daily physical activity and to investigate possible relationships between physical activity levels and clinical outcome. These devices use piezoelectric accelerometers, which measure body's acceleration, in one, two or three axes. The signal can be transformed into an estimate of energy expenditure using one of variety of algorithms, or summarized as activity counts or vector magnitude units (reflecting acceleration). With the information obtained in the vertical plane or through pattern recognition, steps and walking time can also be derived.

An example of a commonly used accelerometer is the multisensory armband device SenseWear^{™} Pro Armband (BodyMedia, Inc., Pittsburg, PA, USA) (Watz H et al, Eur Respir J, 2009; 33: 262-272; Troosters T et al, The Open Respiratory Medicine Journal, 2011, 5, 1-9; O'Donnell D E et al, Respiratory Medicine, 2011, 105, 1030-1036; Waschki B et al, Respiratory Medicine, 2012, 106, 522-530).

Parameters of physical activity over time as determined by an accelerometer, include: 1) average number of steps per day; 2) average time (minutes) spent per day in at least moderate activity (defined as any physical activity > 3 metabolic equivalents); 3) average active energy expenditure (Kcal spend in at least moderate activities); or 4) physical activity level (PAL).

Van Gestel Arnoldus J R et al, PLOS ONE 2012, 2012, vol. 7, no. 11, e48081 reviews whether it is possible to predict daily physical activity in patients with COPD.

Tiotropium is the first long-acting anticholinergic bronchodilator indicated as a maintenance treatment to relieve symptoms of COPD patients. Although tiotropium has been shown to produce sustained improvement in pulmonary function in patients with moderate to severe COPD, tiotropium has not statistically demonstrated improvements in physical activity of patients with COPD versus placebo (Sciurba F C et al, Am J Respir Crit Care Med 183, 2011, A1589).

It has now surprisingly been found that aclidinium significantly increases physical activity in daily life of respiratory patients, increasing thus overall quality of life.

Aclidinium has the chemical name 3(R)-(2-hydroxy-2,2-dithien-2-ylacetoxy)-1-(3-phenoxypropyl)-1-azoniabicyclo[2.2.2] octane and was first disclosed in WO 01/04118. It is a long-acting muscarinic receptor antagonist recently approved by the US Food and Drug Administration (FDA) and the European Medicines Agency (EMA) for the maintenance treatment to relieve respiratory symptoms in patients with COPD. Pharmaceutical compositions comprising aclidinium are described in EP2100598A1 and in EP2100599A1. H. Chrystyn et al, International Journal Of Clinical Practice, 2012, vol. 66, no. 3, 309 - 317 mentions the use of the Genuair^{®} inhaler and Franois Maltais et al, Respiratory Medicine, vol. 105, no. 4, 580 - 587 mentions the use of aclidinium to improve exercise tolerance in patients with moderate to severe COPD. However, until now, there is no disclosure that aclidinium significantly increases physical activity in daily life of respiratory patients. Its effect in improving physical activity is an unexpected finding of this invention having regard to the lack of significant activity of tiotropium, the reference long-acting antimuscarinic drug currently in the market.

### Summary of the Invention

The present invention provides aclidinium, or any of its stereoisomers or mixture of stereoisomers, or a pharmaceutically acceptable salt or solvate thereof, for use in increasing physical activity in daily life in a patient suffering from chronic obstructive pulmonary disease.

Preferably, aclidinium is in the form of a salt with an anion X⁻. Most preferably, the anion X⁻ is bromide.

In another embodiment, aclidinium is administered as a pharmaceutical composition suitable for inhalation, preferably in the form of a dry powder. The composition can be administered by means of any inhaler device, more preferably the Genuair^{®} device.

Typically, a dry powder formulation comprises a pharmaceutically acceptable carrier selected from mono-, di- or polysaccharides and sugar alcohols. Preferably, the carrier is lactose, more preferably lactose monohydrate, even more preferred alpha-lactose monohydrate.

Aclidinium is administered at least once a day, preferably in the morning or in the evening. More preferably aclidinium is administered twice daily, i.e. two oral inhalations per day. In a most preferred embodiment aclidinium is administered twice daily, one in the morning and another one in the evening. In a preferred embodiment, the aclidinium is administered at least during 3 weeks, preferably at least during more than 52 weeks.

The effective dose of aclidinium to be used per inhalation is the equivalent to a metered nominal dose from 100 to 1000 micrograms of aclidinium bromide per inhalation in a dry powder for inhalation, more preferably 200 or 400 micrograms of aclidinium bromide per inhalation. In a most preferred embodiment the effective dose of aclidinium is the equivalent to a metered nominal dose of 400 micrograms of aclidinium bromide per inhalation.

In a particular embodiment, the effective dose of aclidinium to be used per inhalation is the equivalent to a metered nominal dose of 400 micrograms of aclidinium bromide per inhalation and/or a metered nominal dose of 343 micrograms of aclidinium per inhalation.

In another particular embodiment, the effective dose of aclidinium to be used per inhalation is the equivalent to a delivered dose (the dose leaving the mouthpiece of the inhaler device) of 375 micrograms of aclidinium bromide per inhalation and/or a delivered dose of 322 micrograms of aclidinium per inhalation. The delivered dose can be measured using standard techniques known to those skilled in the art.

In another preferred embodiment, aclidinium is co-administered with an additional medication suitable for the treatment of respiratory diseases, selected for example from one or more of the following: corticosteroids, beta-adrenergic agonists, PDE4 inhibitors, antihistamines, anti-lgE antibodies, leukotriene D4 inhibitors, inhibitors of egfr-kinase, p38 kinase inhibitors and/or NK1-receptor antagonists. The additional medications can be present in the same pharmaceutical composition as aclidinium or in separate pharmaceutical compositions. Preferably, the additional medication is selected from corticosteroids, beta-adrenergic agonists and/or PDE4 inhibitors.

The increase by aclidinium of the physical activity in daily life in a patient suffering from chronic obstructive pulmonary disease can be measured by observing the improvement of one or more of the following:
a) average number of steps per day;
b) minutes of moderate activity per day;
c) average active energy expenditure (expressed in Kcal); or
d) physical activity level (PAL).

The physical activity level (PAL) can be obtained by dividing the total daily energy expenditure by whole-night sleeping energy expenditure. A physical activity level ≥ 1.70 defines an active person, 1.40-1.69 defines a predominantly sedentary person, and < 1.40 defines a very inactive person. A person with a physical activity level of 1.2 is usually chair-or bed-bound.

The invention further provides a pharmaceutical composition comprising aclidinium, as defined herein, for use in increasing the physical activity in daily life in a patient suffering from chronic obstructive pulmonary disease.

The invention further provides aclidinium for use as defined above wherein the patent presents a reduced physical activity. Preferably, the reduced physical activity involves one or more of the following:
a) reduced average number of steps per day;
b) reduced minutes of moderate activity per day;
c) reduced average active energy expenditure; or
d) reduced physical activity level (PAL).

### Detailed description of the invention

Typically, aclidinium is administered in the form of a salt with an anion X⁻, wherein X⁻ is a pharmaceutically acceptable anion of a mono or polyvalent acid. More typically, X⁻ is an anion derived from an inorganic acid, such as hydrochloric acid, hydrobromic acid, sulphuric acid and phosphoric acid, or an organic acid such as methanesulphonic acid, acetic acid, fumaric acid, succinic acid, lactic acid, citric acid or maleic acid. Most preferably aclidinium is in the form of aclidinium bromide.

Aclidinium bromide is a white powder with a molecular formula of C₂₆H₃₀NO₄S₂Br and a molecular mass of 564.56. It is very slightly soluble in water and ethanol and sparingly soluble in methanol.

The compound used in the invention may exist in both unsolvated and solvated forms. The term solvate is used herein to describe a molecular complex comprising a compound used in the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds used in the present invention, such as a hydrate.

The words "treatment" and "treating" are to be understood as embracing amelioration of symptoms of a disease or condition and/or elimination or reduction of the cause of the disease or condition and/or prevention of the appearance of the disease or its symptoms.

The term "therapeutically effective amount" refers to an amount sufficient to effect treatment when administered to a patient in need of treatment.

Aclidinium can also be used in combination with other drugs known to be effective in the treatment of the diseases or the disorders indicated above. For example aclidinium can be combined with corticosteroids or glucocorticoids, beta-adrenergic agonists, PDE4 inhibitors, antihistamines, anti-IGE antibodies, leukotriene D4 antagonists, inhibitors of egfr kinase, p38 kinase inhibitors and/or NK-1 receptor agonists.

Corticosteroids that can be combined with aclidinium in the present invention particularly include those suitable for administration by inhalation in the treatment of respiratory diseases or conditions, e.g., prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate. Budesonide, fluticasone propionate and mometasone furoate are especially preferred.

Beta-adrenergic agonists that can be combined with aclidinium in the present invention particularly include β2 adrenergic agonists useful for treatment of respiratory diseases or conditions, for example, selected from the group consisting of arformoterol, bambuterol, bitolterol, broxaterol, carbuterol, clenbuterol, dopexamine, fenoterol, formoterol, hexoprenaline, ibuterol, isoprenaline, mabuterol, meluadrine, nolomirole, orciprenaline, pirbuterol, procaterol, reproterol, ritodrine, rimoterol, salbutamol, salmeterol, sibenadet, sulfonterol, terbutaline, tulobuterol, vilanterol, olodaterol, KUL-1248, abediterol, carmoterol and indacaterol, in free or pharmaceutically acceptable salt form. Preferably, the β2 adrenergic agonist is a long-acting β2 adrenergic agonist, e.g., selected from the group consisting of formoterol, salmeterol, carmoterol, vilanterol, olodaterol, abediterol and indacaterol in free or pharmaceutically acceptable salt form.

PDE4 inhibitors that can be combined with aclidinium in the present invention include denbufylline, rolipram, cipamfylline, arofylline, filaminast, piclamilast, mesopram, drotaverine hydrochloride, lirimilast, roflumilast, cilomilast, 6-[2-(3,4-Diethoxyphenyl)thiazol-4-yl]pyridine-2-carboxylic acid, (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxoacetamide, 9-(2-Fluorobenzyl)-N6-methyl-2-(trifluoromethyl)adenine, N-(3,5-Dichloro-4-pyridinyl)-8-methoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2-methoxyethyl)pyridin-2(1H)-one, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent application numbers WO 03/097613, WO 2004/058729, WO 2005/049581, WO 2005/123692, WO 2005/123693 and WO 2010/069504.

Aclidinium for use in the present invention may be administered by any suitable route to provide local antimuscarinic action. It is preferably administered by inhalation, e.g., as a powder, spray, or aerosol, preferably as a dry powder. Pharmaceutical compositions comprising aclidinium may be prepared using conventional diluents or excipients and techniques known in the galenic art.

Medicaments for administration in a dry powder for inhalation desirably have a controlled particle size. The optimum particle size for inhalation into the bronchial system is usually 1-10 µm, preferably 2-5µm. Particles having a size above 20 µm are generally too large when inhaled to reach the small airways. To achieve these particle sizes the particles of the active ingredient as produced may be size reduced by conventional means, e.g. by micronisation or supercritical fluid techniques. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient, for example a mono-, di- or polysaccharide or sugar alcohol, such as lactose, mannitol or glucose is generally employed. The particle size of the excipient will usually be much greater than the inhaled medicament within the present invention. When the excipient is lactose it will typically be present as lactose particles, preferably crystalline alpha lactose monohydrate, e.g., having an average particle size range of 20-1000 µm, preferably in the range of 90-150 µm. In one embodiment, the lactose particles for use in formulations of the invention have a d10 in the range of 90 - 160 µm, a d50 in the range of 170 - 270 µm, and d90 in the range of 290 - 400 µm.

Suitable lactose materials for use in the present invention are commercially available, e.g., from DMW Internacional (Respitose GR-001, Respitose SV-001, Respitose SV-003); Meggle (Capsulac 60, Inhalac 70, Capsulac 60 INH); and Borculo Domo (Lactohale 100-200, Lactohale 200-300, and Lactohale 100-300).

The ratio between the lactose particles and aclidinium by weight will depend on the inhaler device used, but is typically, e.g., 5:1 to 200:1, preferably 25:1 to 150:1, more preferably 30:1 to 70:1.

In a preferred embodiment, the aclidinium is administered in the form of a dry powder formulation of aclidinium bromide in admixture with lactose, in a ratio by weight of aclidinium to lactose of 1:50 to 1:150, suitable for administration via a dry powder inhaler, wherein the aclidinium particles have an average particle size of from 2 to 5 µm in diameter, e.g., less than 3 µm in diameter, and the lactose particles have have a d10 of 90 - 160 µm, a d50 of 170 - 270 µm, and d90 of 290 - 400 µm.

Dry powder compositions for topical delivery to the lung by inhalation may, for example, be presented in capsules and cartridges of for example gelatine or blisters of for example laminated aluminium foil, for use in an inhaler or insufflator. Each capsule or cartridge may generally contain between 0.001-200 mg, more preferably 0.01-100 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

Aclidinium is preferably administered with a multi-dose inhaler, more preferably with the Genuair^{®} device (formerly known as Novolizer SD2FL), which is described the PCT patent application numbers WO 97/000703, WO 03/000325 and WO 2006/008027 and in Chrystyn H et al, Int J Clin Pract, March 2012, 66, 3, 309-317 (first published online on 16 February 2012).

### Example 1

In a Phase III randomized, double-blind, placebo controlled, 2 period crossover trial, patients with moderate to severe COPD received a dose of aclidinium equivalent to a metered nominal dose of 400 micrograms of aclidinium bromide per inhalation twice-daily (in the morning, 9 am, and in the evening, 9 pm) and placebo for two periods of 3 weeks, with a washout period of 2 weeks between treatment periods.

Patients were randomised to receive either a dose of aclidinium equivalent to a metered nominal dose of 400 micrograms of aclidinium bromide per inhalation twice-daily in the first period followed by placebo in the second period, or to receive placebo in the first period followed by aclidinium bromide 400 micrograms twice-daily in the second period. Both aclidinium bromide and placebo were administered with a Genuair^{®} multidose dry powder inhaler.

Physical activity (as minutes of at least moderate activity per day and as average active energy expenditure expressed in Kcal) was measured in the last week of the treatment with aclidinium and placebo using a multisensor armband (SenseWear^{™} Pro Armband; BodyMedia, Pittsburg, PA, USA), was worn on the upper right over the triceps muscle, according to the method described in Watz B et al, Eur Respir J, 2009; 33: 262-272. The multisensory armband incorporates a biaxial accelerometer that records steps per day and physiological sensors of energy expenditure.

A valid period of measurement was defined as 5 days of measurement with the patients wearing the accelerometer at least 22 hours per day.

The Intention-to-Treat (ITT) population included 109 patients.

Results are displayed in Table 1.

**Table 1. Change from baseline in physical activity parameters**

| | Change from baseline at week 3 | | |
|---|---|---|---|
| Parameter | Placebo (n=83) | Aclidinium (n=85) | Δ Comparison versus placebo |
| Duration of at least moderate activity (minutes) | -5.9 (SE = 4.1) | 4.2 (SE = 4.1) | 10 (p< 0.05) |
| Daily active energy expenditure (Kcal) | -32.7 (SE = 21.0) | 21.9 (SE = 20.8) | 55 (p< 0.05) |

| | | | |
|---|---|---|---|
| n=number of patients in the analysis SE=standard error | | | |

As it can be observed in Table 1, after 3 weeks of treatment, 400 micrograms of aclidinium bromide per inhalation twice-daily showed statistically significant increases in the adjusted mean change from baseline compared to placebo in the duration of at least moderate activity (10 minutes; p< 0.05 versus placebo). Moderate activity defined as any physical activity > 3 metabolic equivalents.

Aclidinium bromide also showed statistically significant increases in the adjusted mean change from baseline compared to placebo in daily active energy expenditure activity (55 Kcal; p< 0.05 versus placebo)

These phase III results demonstrate a remarkable improvement in physical activity produced by aclidinium, which was not observed with tiotropium, the reference standard in COPD treatment, in previous trials (Sciurba F C et al, Am J Respir Crit Care Med 183, 2011, A1589).

## Claims

1. Aclidinium or any of its stereoisomers or mixture of stereoisomers, or a pharmaceutically acceptable salt or solvate thereof, for use in increasing physical activity in daily life in a patient suffering from chronic obstructive pulmonary disease.

2. Aclidinium for use according to claim 1, wherein aclidinium is in the form of aclidinium bromide.

3. Aclidinium for use according to claim 1 or 2, wherein aclidinium is in the form of a dry powder formulation suitable for inhalation.

4. Aclidinium for use in a dry powder formulation according to claim 3, providing a metered nominal dose of aclidinium equivalent to from 100 to 1000 micrograms of aclidinium bromide per inhalation; preferably 200 or 400 micrograms of aclidinium bromide.

5. Aclidinium for use in a dry powder formulation according to claim 3, providing a metered nominal dose of aclidinium equivalent to 400 micrograms of aclidinium bromide per inhalation and/or a metered nominal dose of 343 micrograms of aclidinium per inhalation.

6. Aclidinium for use in a dry powder formulation according to claim 3, providing a delivered dose of aclidinium equivalent to 375 micrograms of aclidinium bromide per inhalation and/or a delivered dose of 322 micrograms of aclidinium per inhalation.

7. Aclidinium for use according to any preceding claim, wherein aclidinium is administered one or more times per day, preferably wherein aclidinium is administered twice daily.

8. Aclidinium for use according to any preceding claim, wherein aclidinium is co-administered with a therapeutically effective amount of a corticosteroid, a beta-adrenergic agonist and/or a PDE4 inhibitor.

9. Aclidinium for use according to any of the preceding claims, wherein the physical activity in daily life is increased by increasing one or more of the following:
a) average number of steps per day;
b) minutes of moderate activity per day;
c) average active energy expenditure; or
d) physical activity level.

10. A pharmaceutical composition comprising aclidinium, as defined in any preceding claim, for use in increasing physical activity in daily life in a patient suffering from chronic obstructive pulmonary disease.

11. Aclidinium for use as defined in claim 1 or 2 wherein the patient presents a reduced physical activity.

12. Aclidinium for use according to claim 11, wherein the reduced physical activity involves one or more of the following:
a) reduced average number of steps per day;
b) reduced minutes of moderate activity per day;
c) reduced average active energy expenditure; or
d) reduced physical activity level (PAL).

## Patentansprüche

1. Aclidinium oder eines seiner Stereoisomere oder eine Mischung von Stereoisomeren oder ein pharmazeutisch unbedenkliches Salz oder Solvat davon für die Verwendung zum Steigern der körperlichen Aktivität im täglichen Leben eines Patienten, der an einer chronisch obstruktiven Lungenerkrankung leidet.

2. Aclidinium für die Verwendung nach Anspruch 1, wobei Aclidinium in der Form von Aclidiniumbromid vorliegt.

3. Aclidinium für die Verwendung nach Anspruch 1 oder 2, wobei Aclidinium in der Form einer für die Inhalation geeigneten Trockenpulverformulierung vorliegt.

4. Aclidinium für die Verwendung in einer Trockenpulverformulierung nach Anspruch 3, die eine dosierte Nenndosis von Aclidinium bereitstellt, die 100 bis 1000 Mikrogramm Aclidiniumbromid pro Inhalation entspricht; vorzugsweise 200 oder 400 Mikrogramm Aclidiniumbromid.

5. Aclidinium für die Verwendung in einer Trockenpulverformulierung nach Anspruch 3, die eine dosierte Nenndosis von Aclidinium, die 400 Mikrogramm Aclidiniumbromid pro Inhalation entspricht, und/oder eine dosierte Nenndosis von 343 Mikrogramm Aclidinium pro Inhalation bereitstellt.

6. Aclidinium für die Verwendung in einer Trockenpulverformulierung nach Anspruch 3, die eine abgegebene Dosis von Aclidinium, die 375 Mikrogramm Aclidiniumbromid pro Inhalation entspricht, und/oder eine abgegebene Dosis von 322 Mikrogramm Aclidinium pro Inhalation bereitstellt.

7. Aclidinium für die Verwendung nach einem der vorhergehenden Ansprüche, wobei Aclidinium ein- oder mehrmals täglich verabreicht wird, vorzugsweise wobei Aclidinium zweimal täglich verabreicht wird.

8. Aclidinium für die Verwendung nach einem der vorhergehenden Ansprüche, wobei Aclidinium zusammen mit einer therapeutisch wirksamen Menge eines Kortikosteroids, eines beta-adrenergen Agonisten und/oder eines PDE4-Inhibitors verabreicht wird.

9. Aclidinium für die Verwendung nach einem der vorhergehenden Ansprüche, wobei die körperliche Aktivität im täglichen Leben durch das Steigern einer/eines oder mehrerer der Folgenden gesteigert wird:
a) durchschnittlicher Anzahl von Schritten pro Tag;
b) Minuten moderater Aktivität pro Tag;
c) durchschnittlicher aktiver Energieaufwand; oder
d) Leistungsumsatz.

10. Pharmazeutische Zusammensetzung, umfassend Aclidinium nach einem der vorhergehenden Ansprüche für die Verwendung zum Steigern der körperlichen Aktivität im täglichen Leben bei einem Patienten, der an einer chronisch obstruktiven Lungenerkrankung leidet.

11. Aclidinium für die Verwendung nach Anspruch 1 oder 2, wobei der Patient eine reduzierte körperliche Aktivität aufweist.

12. Aclidinium für die Verwendung nach Anspruch 11, wobei die reduzierte körperliche Aktivität eine/einen oder mehrere der Folgenden einschließt:
a) reduzierte durchschnittliche Anzahl von Schritten pro Tag;
b) reduzierte Minuten moderater Aktivität pro Tag
c) reduzierten durchschnittlichen aktiven Energieaufwand; oder
d) reduzierten Leistungsumsatz (*physical activity level* - PAL).

## Revendications

1. Aclidinium ou un quelconque de ses stéréoisomères ou mélange de stéréoisomères, ou un sel ou solvate pharmaceutiquement acceptable de celui-ci, pour l'utilisation dans l'augmentation d'activité physique dans la vie quotidienne d'un patient souffrant de maladie pulmonaire obstructive chronique.

2. Aclidinium pour l'utilisation selon la revendication 1, dans lequel l'aclidinium est sous la forme de bromure d'aclidinium.

3. Aclidinium pour l'utilisation selon la revendication 1 ou 2, dans lequel l'aclidinium est sous la forme d'une préparation en poudre sèche appropriée pour l'inhalation.

4. Aclidinium pour l'utilisation dans une préparation en poudre sèche selon la revendication 3, fournissant une dose nominale d'aclidinium équivalente à 100 à 1000 microgrammes de bromure d'aclidinium par inhalation ; de préférence 200 ou 400 microgrammes de bromure d'aclidinium.

5. Aclidinium pour l'utilisation dans une préparation en poudre sèche selon la revendication 3, fournissant une dose nominale d'aclidinium équivalente à 400 microgrammes de bromure d'aclidinium par inhalation et/ou une dose nominale de 343 microgrammes d'aclidinium par inhalation.

6. Aclidinium pour l'utilisation dans une préparation en poudre sèche selon la revendication 3, fournissant une dose administrée d'aclidinium équivalente à 375 microgrammes de bromure d'aclidinium par inhalation et/ou une dose administrée de 322 microgrammes d'aclidinium par inhalation.

7. Aclidinium pour l'utilisation selon une quelconque revendication précédente , dans lequel l'aclidinium est administré une ou plusieurs fois par jour, de préférence dans lequel l'aclidinium est administré deux fois par jour.

8. Aclidinium pour l'utilisation selon une quelconque revendication précédente, dans lequel l'aclidinium est administré conjointement avec une quantité thérapeutiquement efficace d'un corticostéroïde, d'un agoniste bêta-adrénergique et/ou d'un inhibiteur de PDE4.

9. Aclidinium pour l'utilisation selon l'une quelconque des revendications précédentes, dans lequel l'activité physique dans la vie quotidienne est augmentée en augmentant un ou plusieurs de ce qui suit :
a) nombre moyen de pas par jour ;
b) minutes d'activité modérée par jour ;
c) dépense énergétique active moyenne ; ou
d) niveau d'activité physique.

10. Composition pharmaceutique comprenant de l'aclidinium, selon une quelconque revendication précédente, pour l'utilisation dans l'augmentation d'activité physique dans la vie quotidienne d'un patient souffrant de maladie pulmonaire obstructive chronique.

11. Aclidinium pour l'utilisation selon la revendication 1 ou 2, dans lequel le patient a une activité physique réduite.

12. Aclidinium pour l'utilisation selon la revendication 11, dans lequel l'activité physique réduite implique un ou plusieurs de ce qui suit :
a) nombre moyen réduit de pas par jour ;
b) minutes réduites d'activité modérée par jour ;
c) dépense énergétique active moyenne réduite ; ou
d) niveau d'activité physique (NAP) réduit.
